# EUROPEAN PATENT APPLICATION

(11) **EP 4 365 205 A1**
(43) Date of publication of application: **08.05.2024**
(21) Application number: 22831776.4
(22) Date of filing: 21.06.2022
(51) Int. Cl.: C07K 16/46, C07K 16/28, C07K 19/00, C12N 15/13, C12N 15/63, C12P 21/08, A61K 39/395, A61K 47/68, A61P 35/00

(54) **TRISPECIFIC ANTIBODY AND PREPARATION METHOD THEREFOR AND USE THEREOF**

(30) Priority: 29.06.2021 CN 202110726683
(71) Applicant: Sunshine Guojian Pharmaceutical (Shanghai) Co., Ltd., Shanghai 201203 (CN)
(72) Inventor: ZHAO, Jie, Shanghai 201203 (CN); HUANG, Haomin, Shanghai 201203 (CN); ZHU, Zhenping, Shanghai 201203 (CN)
(74) Representative: reuteler & cie SA
(86) International application number: PCT/CN2022/100105
(87) International publication number: WO 2023/273958

(57) **Abstract**

Provided is a hexavalent trispecific antibody. The hexavalent trispecific antibody is a dimer formed by two monomers; each monomer comprises a heavy chain, a first light chain, and a second light chain; from N terminal to C terminal, the heavy chain comprises an antibody heavy chain variable region element Z1 for a first target, an antibody heavy chain variable region element Z2 for a second target, and an antibody heavy chain variable region element Z3 and an antibody heavy chain constant region Z4 for a third target which are connected in series; from the N terminal to the C terminal, the first light chain comprises an antibody light chain variable region element Z5 for the first target and an antibody light chain variable region element Z6 for the second target which are connected in series; from the N terminal to the C terminal, the second light chain comprises an antibody light chain variable region element Z7 and a light chain constant region Z8 for the third target, which are connected in series; and the first light chain and the second light chain are respectively combined with the heavy chain, so that the hexavalent trispecific antibody specifically binds to the first target, the second target, and the third target. The first target, the second target, and the third target can be PD-1, HER-2, and LAG-3.

## Description

### Technical field

The present invention belongs to the field of antibodies, and specifically relates to a trispecific antibody and the preparation method and application thereof.

### Background

The anti-PD-1/PD-L1 monoclonal antibody is the earliest widely used cancer immunotherapy. Subsequently, bispecific antibodies emerged as cancer immunotherapy, where one end of these antibodies can bind to antigens on the surface of cancer cells, while the other end can bind to CD3 on the surface of T cells, recruiting and activating T cells to kill cancer cells (such as Amgen's Blinatumomab). Blinatumomab is a bispecific antibody that targets both the CD19 antigen on the surface of cancer cells and the CD3 receptor on the surface of T cells. It can double the response rate and survival time of patients with advanced B-cell acute lymphoblastic leukemia (B-ALL). These bispecific antibodies can effectively activate the anticancer activity of T cells.

However, typically when T cells are activated, not only the CD3 mediated si gnaling pathway is activated, but a co stimulatory receptor mediated signaling path way called CD28 is also activated. This dual activation can ensure that T cells m aintain longer anti-tumor activity. In a recent study published in the journal Nature Cancer (reference: Wu et al., (2019)) Trispecific antibiotics enhance the therapeuti c efficacy of tutor directed T cells through T cell receiver co stimulation Nature Cancer, https://doi.org/10.1038/s43018-019-0004-z) Sanofi's R&D team has develope d a trispecific antibody that not only binds to tumor associated antigens and T cel l CD3, but also to CD28 on the surface of T cells, thereby enhancing their antica ncer activity. Activating CD28 receptors can stimulate the expression of Bcl xL pr otein, which can prevent T cell apoptosis and prolong T cell activity. CD28 has b een used in CAR-T therapy as a co-stimulatory receptor. Some CAR-T cells expre ss receptors that contain the co-stimulatory protein domains of CD3 and CD28. *In vitro* experiments, compared to the approved anti-CD38 monoclonal antibody Dara tumumab, the proportion of cancer cells lysed by trispecific antibodies increased b y 3-4 times. In a mouse model of multiple myeloma, the tri-specific antibody was also able to reduce the size of transplanted tumors in a dose-dependent manner.

Researchers still need to test the safety of this innovative antibody. A common side effect of cancer immunotherapy that stimulates T cell activity is cytokine release syndrome, which is caused by excessive activation of T cells. In non-human primate models, this trispecific therapy exhibits controllable safety, but its safety needs to be validated in both humans and MM (multiple myeloma) patients.

The review, published in Nature (reference: Garfall and June. (2019) Trispecific antibodies offer a third way forward for antagonists alike Nature, doi: 10.1038/d41586-019-03495-3) , said patients with multiple myeloma have been in need of new treatments because even effective ones like CAR-T only provide temporary relief for most patients. Triple specific antibodies may provide a flexible platform for delivering precise immune regulatory signal combinations based on the tumor microenvironment, making it safer and more effective than combination therapies composed of triple single specific antibodies.

In addition, Numab has disclosed its trispecific antibody design method in US20180355024A1, which is characterized by a plurality of fragments of ScFv and FV. Xencor Company has disclosed the design method of its triple specific antibody in US20190352416A1, which is characterized by the use of heavy chain heterodimerization technology and ScFv fragments.

In addition to the products and companies mentioned above, several other early studies have been published in internationally renowned journals. In 2017, a study published in *Science* (reference: Liang Xu et al.Trispecific broadly neutralizing HIV antibodies mediate potent SHIV protection in Macaques. Science, Published online: 20 Sep 2017, doi: 10.1126 / Science aan8630.) shows that scientists in the laboratory has developed a trispecific antibody. It protects monkeys from two strains of human-monkey chimeric immunodeficiency virus (SHIV). The trispecific antibody combines a unique structure that broadly neutralizes the HIV antibodies VRC01, PGDM1400 and 10E8v4, three HIV binding fragments derived from three natural antibodies, each of which strongly neutralizes many HIV strains. Using trispecific antibodies to treat cancer would be a very important conceptual breakthrough. Since trispecific antibodies are a flexible carrier that can specifically deliver combinations of immunomodulatory signals in the tumor microenvironment, this may be safer and more effective than combinations of multiple specific immunomodulatory monoantibodies. Therefore, this combination strategy is expected to improve the accuracy and efficacy of current immunotherapies and further expand the scope of immunotherapy application. Trispecific antibodies can simultaneously bind to three different targets, which is expected to provide a strong foundation for the development of treatment methods for HIV infection, other infectious diseases, autoimmune diseases, and cancer.

Therefore, it is necessary to develop new and effective trispecific antibodies in this field.

### Summary of the invention

The purpose of the present invention is to provide trispecific antibodies, a preparation method and a use thereof.

In the first aspect of the invention, a hexavalent trispecific antibody is provided. The hexavalent trispecific antibody is a dimer formed by two monomers, and each monomer comprises a heavy chain, a first light chain and a second light chain;

The heavy chain comprises an antibody heavy chain variable region element Z1 for a first target, an antibody heavy chain variable region element Z2 for a second target, an antibody heavy chain variable region element Z3 for a third target, and a antibody heavy chain constant region Z4 in tandem from the N end to the C end.

The first light chain and the second light chain respectively cooperate with the heavy chain, so that the hexavalent trispecific antibody specifically binds to the first target, second target, and third target.

In another preferred example, the first light chain includes an antibody light chain variable region element Z5 for the first target and an antibody light chain variable region element Z6 for the second target in tandem from the N to the C end.

In another preferred example, the second light chain comprises an antibody light chain variable region element Z7 for the third target and an antibody light chain constant region Z8 in tandem from the N end to the C end.

In another preferred example, the antibody heavy chain constant region Z4 comprises the CH1, CH2, and CH3 regions.

In another preferred example, the first light chain cooperates with the antibody heavy chain variable region element Z1 and the antibody heavy chain variable region element Z2; and the second light chain cooperates with the antibody heavy chain variable region element Z3.

In another preferred example, the second light chain further cooperates with the CH1 region in the antibody heavy chain constant region Z4.

In another preferred example, the antibody heavy chain constant region Z4 is derived from an antibody targeting the first target, antibody targeting the second target, or antibody targeting the third target.

In another preferred example, the antibody heavy chain variable region element Z1, antibody heavy chain variable region element Z2, antibody heavy chain variable region element Z3, and antibody heavy chain constant region Z4 are each independently whole human or humanized.

In another preferred example, the antibody light chain variable region element Z5, the antibody light chain variable region element Z6, the antibody light chain variable region element Z7, and the antibody light chain constant region Z8 are independently full-human or humanized.

In another preferred example, the two adjacent elements in the antibody heavy chain variable region element Z1, antibody heavy chain variable region element Z2, antibody heavy chain variable region element Z3, and antibody heavy chain constant region Z4 can be directly connected or connected through a linker.

In another preferred example, the antibody light chain variable region element Z5 and the antibody light chain variable region element Z6 may be connected directly or through a linker.

In another preferred example, the antibody light chain variable region element Z7 and the antibody light chain constant region Z8 may be connected directly or through a linker.

In another preferred example, the linker comprises a flexible linker and a rigid linker.

In another preferred example, the linker is a peptide linker of 1-35 amino acids in length, and preferably a peptide linker of 6-30 amino acids in length.

In another preferred example, the first target, second target, and third target are PD-1, HER-2, and LAG-3.

In another preferred example, the first target is PD-1, the second target is HER-2, and the third target is LAG-3.

In another preferred example, the first target is HER-2, the second target is PD-1, and the third target is LAG-3.

In another preferred example, the first target is PD-1, the second target is LAG-3, and the third target is HER-2.

In another preferred example, the first target is LAG-3, the second target is PD-1, and the third target is HER-2.

In another preferred example, the first target is HER-2, the second target is LAG-3, and the third target is PD-1.

In another preferred example, the first target is LAG-3, the second target is HER-2, and the third target is PD-1.

In another preferred example, the hexavalent trispecific antibody comprises a heavy chain, a first light chain, and a second light chain; wherein each heavy chain has the structure of equation I:

Z1-Z2-Z3-Z4 (I)

Z1 is a heavy chain variable region element for the first target;
Z2 is a heavy chain variable region element for the second target.
Z3 is a heavy chain variable region element for the third target;
Z4 is the constant heavy chain region CH1, CH2 and CH3.
"-" is independently a bond or a linker;
and the first light chain and the second light chain respectively cooperates with the heavy chain, so that the hexavalent trispecific antibody specifically binds to the first target, the second target, and the third target.

In another preferred example, the first light chain cooperates with Z1 and Z2, while the second light chain cooperates with Z3.

In another preferred example, the first light chain is also connected to Z2 through a disulfide bond.

In another preferred example, the second light chain cooperates with Z3 and CH1, and the second light chain is also connected to CH1 through a disulfide bond.

In another preferred embodiment, the first light chain has a structure shown in formula II:

Z5-Z6 (II);

wherein Z5 is a light chain variable region element for the first target; Z6 is a light chain variable region element for the second target.

In another preferred embodiment, the second light chain has a structure shown in formula III:

Z7-Z8 (III);

wherein Z7 is a light chain variable region element for the third target, and Z8 is a light chain constant region.

In another preferred example, the first target is PD-1, the second target is HER-2, and the third target is LAG-3.

In another preferred example, the first target is HER-2, the second target is PD-1, and the third target is LAG-3.

In another preferred example, the first target is PD-1, the second target is LAG-3, and the third target is HER-2.

In another preferred example, the first target is LAG-3, the second target is PD-1, and the third target is HER-2.

In another preferred example, the first target is HER-2, the second target is LAG-3, and the third target is PD-1.

In another preferred example, the first target is LAG-3, the second target is HER-2, and the third target is PD-1.

In another preferred embodiment, the first light chain has an amino acid sequence selected from the following group: SEQ ID NO: 15, 17, or 19.

In another preferred embodiment, the second light chain has an amino acid sequence as shown in SEQ ID NO: 13.

In another preferred embodiment, the heavy chain has an amino acid sequence selected from the following group: SEQ ID NO: 14, 16, or 18.

In another preferred embodiment, the hexavalent trispecific antibody is a dimer.

In another preferred embodiment, the hexavalent trispecific antibody is a homologous or heterodimer.

In another preferred example, the hexavalent trispecific antibody comprises two monomers, each containing a heavy chain, a first light chain, and a second light chain, wherein each monomer has the following formula IV structure: 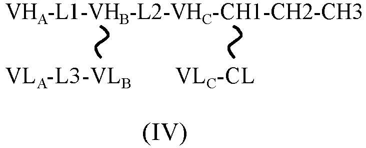 wherein,
VH_{A}-L1-VH_{B}-L2-VH_{C}-CH1-CH2-CH3 is the heavy chain;
VL_{A}-L3-VL_{B} is the first light chain;
VLc-CL is the second light chain;
VH_{A} is the heavy chain variable region for the first target;
VH_{B} is the heavy chain variable region for the second target;
VHc is the heavy chain variable region for the third target;
CH1, CH2 and CH3 are the constant heavy chain regions CH1, CH2 and CH3, respectively;
VL_{A} is the light chain variable region for the first target;
VL_{B} is the light chain variable region for the second target;
VLc is the light chain variable region for the third target;
CL is the light chain constant region of the antibody;
L1, L2, and L3 are independently linkers;
each "-" is independently a bond;
"~" represents a disulfide bond or covalent bond;
wherein the hexavalent trispecific antibody binds simultaneously to the first target, the second target, and the third target.

In another preferred example, the first target is HER-2, the second target is PD-1, and the third target is LAG-3.

In another preferred example, the first target is PD-1, the second target is LAG-3, and the third target is HER-2.

In another preferred example, the first target is LAG-3, the second target is PD-1, and the third target is HER-2.

In another preferred example, the first target is HER-2, the second target is LAG-3, and the third target is PD-1.

In another preferred example, the first target is LAG-3, the second target is HER-2, and the third target is PD-1.

In another preferred example, the hexavalent trispecific antibody comprises two monomers, each containing a heavy chain, a first light chain, and a second light chain, wherein each monomer has the structure of following formula IV: 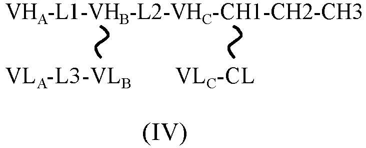 wherein,
VH_{A}-L1-VH_{B}-L2-VH_{C}-CH1-CH2-CH3 is the heavy chain;
VL_{A}-L3-VL_{B} is the first light chain;
VL_{C}-CL is the second light chain;
VH_{A} is the heavy chain variable region of an anti-PD-1 antibody;
VH_{B} is the heavy chain variable region of an anti-HER-2 antibody;
VHc is the heavy chain variable region of an anti-LAG-3 antibody;
CH1, CH2 and CH3 are the constant heavy chain regions CH1, CH2 and CH3, respectively;
VL_{A} is the light chain variable region of an anti-PD-1 antibody;
VL_{B} is the light chain variable region of an anti-HER-2 antibody;
VL_{C} is the light chain variable region of an anti-LAG-3 antibody;
CL is the light chain constant region of the antibody;
L1, L2, and L3 are independently linkers;
each "-" is independently a bond;
"~" represents a disulfide bond or covalent bond;
wherein the hexavalent trispecific antibody binds simultaneously to PD-1, HER-2 and LAG-3.

In another preferred embodiment, the linker is a flexible linking peptide.

In another preferred embodiment, the flexible peptide linker comprises 6-30 amino acids and preferably 10-25 amino acids.

In another preferred embodiment, the flexible linker comprises 2 to 6 G4S.

In another preferred embodiment, the L1, L2, or L3 are each independently 2-4 G4S.

In another preferred embodiment, the heavy chain constant region is the heavy chain constant region of, or derived from, an IgG1, IgG2, IgG3 or IgG4 antibody.

In another preferred embodiment, the heavy chain constant region is selected from the heavy chain constant region of human IgG1 or human IgG4.

In another preferred embodiment, the heavy chain constant region of the human IgG4 comprises the S228P mutation.

In another preferred embodiment, the light chain constant region is the light chain constant region of, or derived from, an IgG1, IgG2, IgG3 or IgG4 antibody.

In another preferred embodiment, the light chain constant region is selected from the light chain constant region of human IgG1 or human IgG4.

In another preferred example, the hexavalent trispecific antibody comprises two heavy chains, two first light chains and two second light chains,
wherein the heavy chain is selected from the following group: a heavy chain, the amino acid sequence of which is shown in SEQ ID NO: 14, 16 or 18;
the first light chain is selected from the following group: a first light chain, the amino acid sequence of which is shown in SEQ ID NO: 15, 17 or 19; and/or
the second light chain is selected from the following group: a second light chain, the amino acid sequence of which is shown in SEQ ID NO: 13.

In another preferred embodiment, the amino acid sequences of the heavy chain, the first light chain and the second light chain in the hexavalent trispecific antibody are shown as SEQ ID No: 14, 15 and 13, respectively.

In another preferred embodiment, the amino acid sequences of the heavy chain, the first light chain and the second light chain in the hexavalent trispecific antibody are shown as SEQ ID No: 16, 17 and 13, respectively.

In another preferred embodiment, the amino acid sequences of the heavy chain, the first light chain and the second light chain in the hexavalent trispecific antibody are shown as SEQ ID No: 18, 19 and 13, respectively.

In a second aspect of the invention, it provides an isolated nucleic acid molecule, encoding the hexavalent trispecific antibody of the the first aspect of the invention.

In another preferred example, the nucleic acid molecule encodes the heavy chain and the first light chain and the second light chain respectively.

In the third aspect of the present invention, it provides an expression vector comprising the nucleic acid molecule of the second aspect of the present invention.

In the fourth aspect of the present invention, it provides a host cell comprising the expression vector of the third aspect of the present invention.

In a fifth aspect of the invention, it provides a preparation method for the hexavalent trispecific antibody described in the first aspect of the invention, which comprises following steps:
(a) culturing the host cell according to the fourth aspect of the present invention to express the hexavalent trispecific antibody;
(b) separating and purifying the hexavalent trispecific antibody described in (a).

In the sixth aspect of the invention, a pharmaceutical composition containing a hexavalent trispecific antibody and a pharmaceutically acceptable carrier as described in the first aspect of the invention is provided.

In another preferred embodiment, the pharmaceutical composition further comprises an anti-tumor agent.

In another preferred embodiment, the pharmaceutical composition is in a unit dosage form.

In another preferred example, the anti-tumor agent may exist separately in a separate package with the trispecific antibody, or the anti-tumor agent may be coupled with the trispecific antibody.

In another preferred example, the dosage form of the pharmaceutical composition includes a dosage form for gastrointestinal or parenteral administration.

In another preferred example, the parenteral administration dosage forms include intravenous, intravenous, subcutaneous, local, intramuscular, intratumoral, intraperitoneal, intracranial, or intracavitary injection.

In the seventh aspect of the invention, it provides that a use of a hexavalent trispecific antibody as described in the first aspect of the invention or a pharmaceutical composition as described in the sixth aspect of the invention in the preparation of a drug for the treatment of a cancer.

In another preferred example, the cancer is selected from the following group: melanoma, renal cancer, prostate cancer, pancreatic cancer, breast cancer, colon cancer, lung cancer, esophageal cancer, head and neck squamous cell cancer, liver cancer, ovarian cancer, cervical cancer, thyroid cancer, glioblastoma, glioma and other vegetative malignant diseases.

In the eighth aspect of the invention, a method for the treatment of a cancer is provided, including the administration of a hexavalent trispecific antibody as described in the first aspect of the invention, or its immune conjugate, or a pharmaceutical composition as described in the sixth aspect of the invention, to a subject in need thereof.

In another preferred example, the cancer is selected from the following groups: melanoma, renal cancer, prostate cancer, pancreatic cancer, breast cancer, colon cancer, lung cancer, esophageal cancer, head and neck squamous cell cancer, liver cancer, ovarian cancer, cervical cancer, thyroid cancer, glioblastoma, glioma and other vegetative malignant diseases.

In the ninth aspect of the invention, an immune conjugate is provided, which comprises:
(a) hexavalent trispecific antibody according to the first aspect of the present invention; and
(b) a coupling moiety selected from the group consisting of a detectable label, drug, toxin, cytokine, radionuclide, or enzyme.

In another preferred embodiment, the coupling moiety is selected from fluorescent or luminescent markers, radiolabels, MRI(magnetic resonance imaging) or CT(computerized X-ray tomography) contrast agents, or enzymes, radionuclides, biotoxins, cytokines capable of producing detectable products.

In another preferred embodiment, the immune conjugate comprises an antibody-drug conjugate (ADC).

In another preferred embodiment, the immune conjugate is used to prepare a drug for preventing and treating tumors.

It should be understood that within the scope of the present invention, the various technical features of the present invention above and the various technical features specifically described hereinafter (as in the embodiments) may be combined with each other to constitute a new or preferred technical solution. Due to space limitations, it is not repeated here.

### Description of the figures

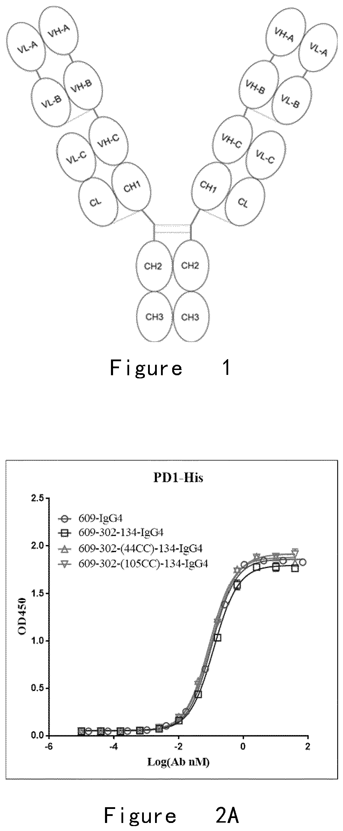

Figure 1 is a structural diagram of the trispecific antibody of the invention, wherein VH-A represents the heavy chain variable region of the first antibody, VH-B represents the heavy chain variable region of the second antibody, and VH-C represents the heavy chain variable region of the third antibody; VL-A represents the light chain variable region of the first antibody, VL-B represents the light chain variable region of the second antibody, and VL-C represents the light chain variable region of the third antibody. CH1, CH2, and CH3 are the three structural domains of the heavy chain constant region, while CL is the light chain constant region. The line segment between the two heavy chains represents the disulfide bond, and the line segment between the heavy and light chains also represents the disulfide bond, wherein the line segment between VH-B and VL-B represents the artificially designed disulfide bond. The line segments between VH-A and VH-B, VH-B and VH-C, and VL-A and VL-B represent artificially designed linkers, and the line segments between CH1 and CH2 represent antibody natural linkers and hinge regions (if the heavy chain is the human IgG4 subtype, the hinge region will contain S228P point mutations).

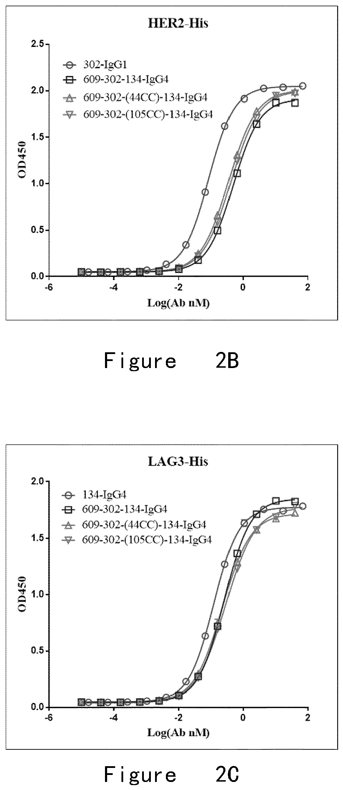

Figure 2 shows the ELISA results of the trispecific antibodies of the present invention. Among them, Figures 2A, 2B, and 2C respectively show the affinity results of the trispecific antibodies of the present invention for PD-1, HER-2, and LAG-3 determined by ELISA.

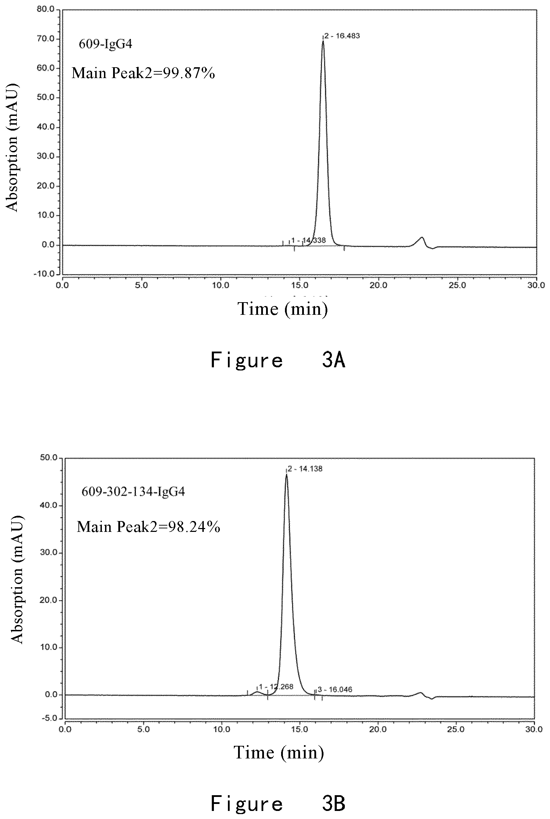

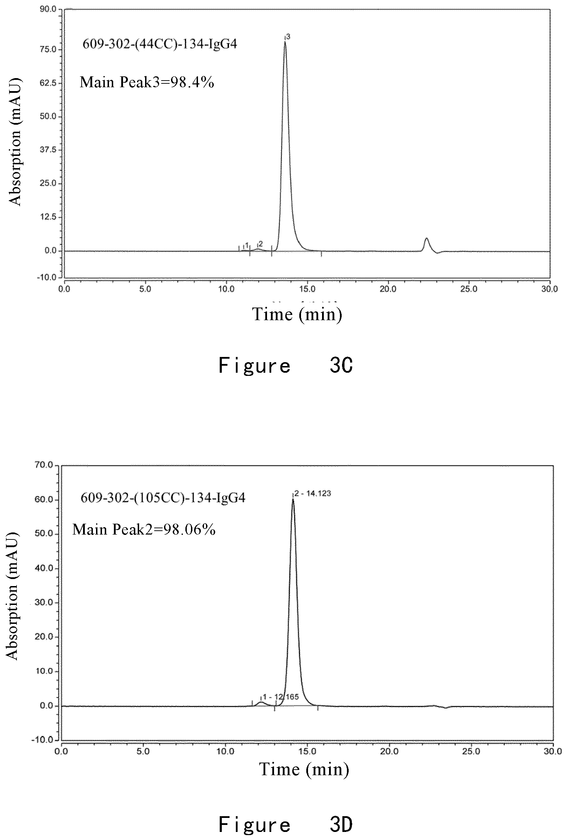

Figure 3 shows the HPLC-SEC profile of monoclonal antibody 609-IgG4 and the trispecific antibody of the present invention. Among them, Figure 3A shows the HPLC-SEC profile of monoclonal antibody 609-IgG4; Figure 3B shows the HPLC-SEC spectrum of 609-302-134 IgG4; Figure 3C shows the HPLC-SEC spectrum of 609-302-(44CC) -134-IgG4; Figure 3D shows the HPLC-SEC spectrum of 609-302- (105CC) -134-IgG4.

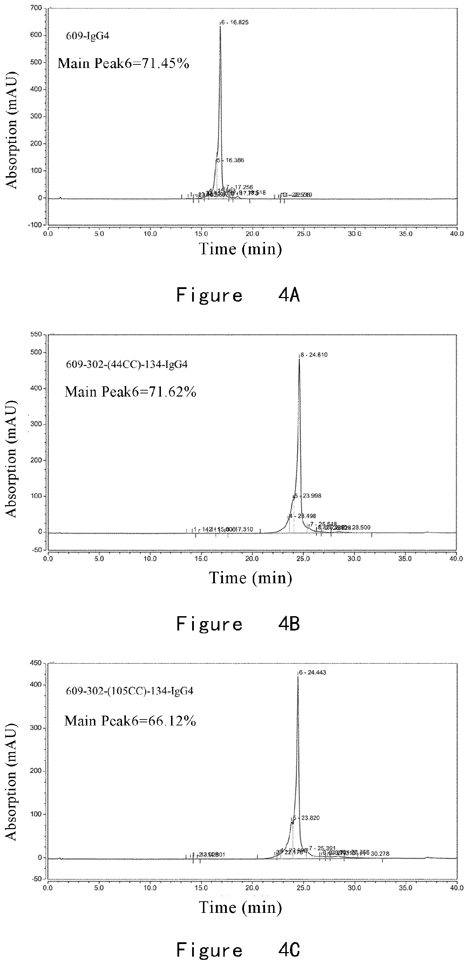

Figure 4 shows the HPLC-IEC profile of monoclonal antibody 609-IgG4 and the trispecific antibody of the present invention. Among them, Figure 4A shows the HPLC-IEC spectrum of monoclonal antibody 609-IgG4; Figure 4B shows the HPLC-IEC spectrum of 609-302- (44CC) -134-IgG4; Figure 4C shows the HPLC-IEC spectrum of 609-302- (105CC) -134-IgG4.

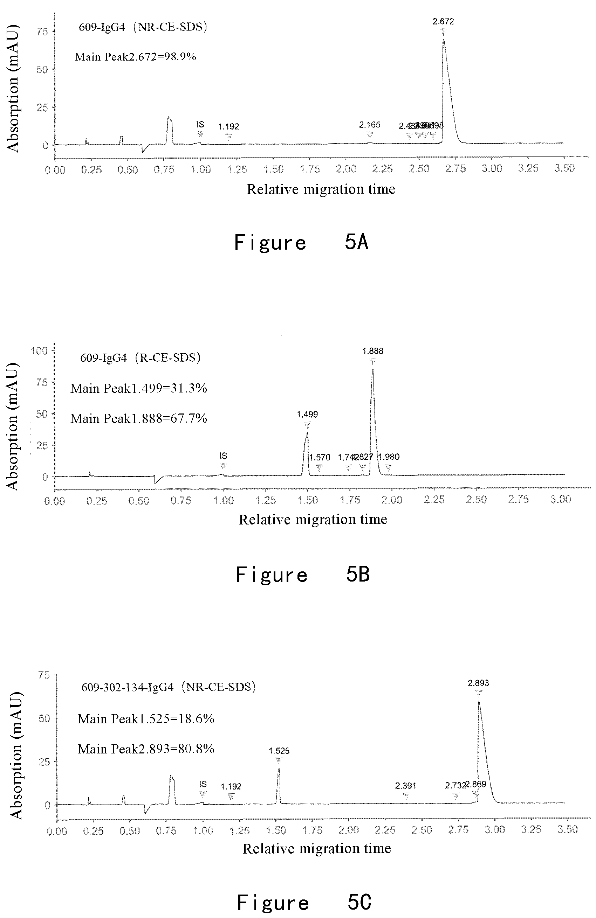

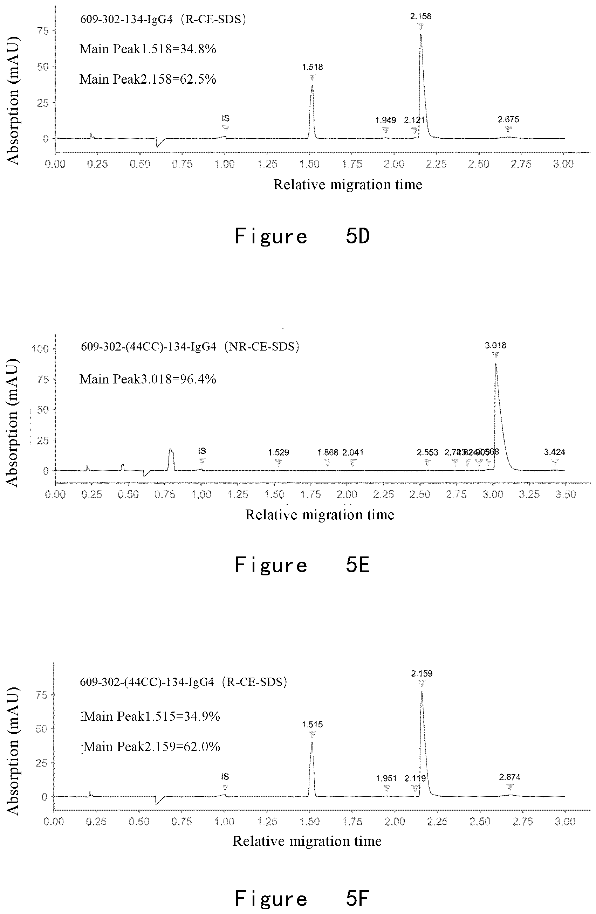

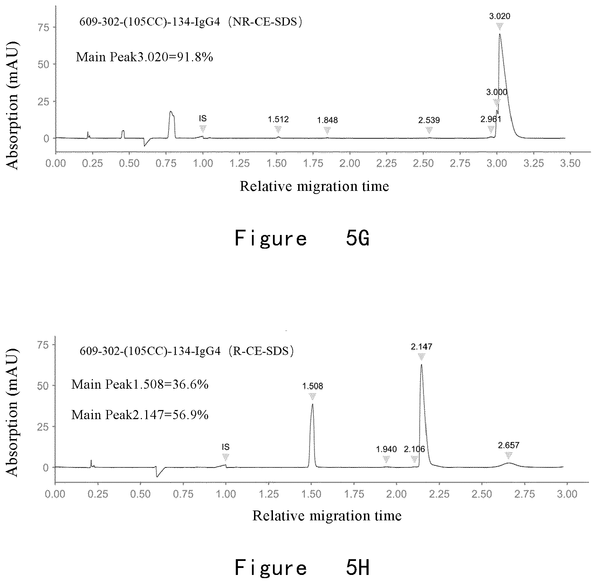

Figure 5 shows the HPLC-IEC profile of monoclonal antibody 609-IgG4 and the trispecific antibody of the present invention. Among them, Figure 5A and Figure 5B represent the NR-CE-SDS and R-CE-SDS spectrum of monoclonal antibody 609-IgG4, respectively; Figures 5C and 5D represent the NR-CE-SDS and R-CE-SDS spectrum of 609-302-134 IgG4, respectively; Figures 5E and 5F represent the NR-CE-SDS and R-CE-SDS spectrum of 609-302- (44CC) -134-IgG4, respectively; Figures 5G and 5H represent the NR-CE-SDS and R-CE-SDS spectrum of 609-302- (105CC) -134-IgG4, respectively.

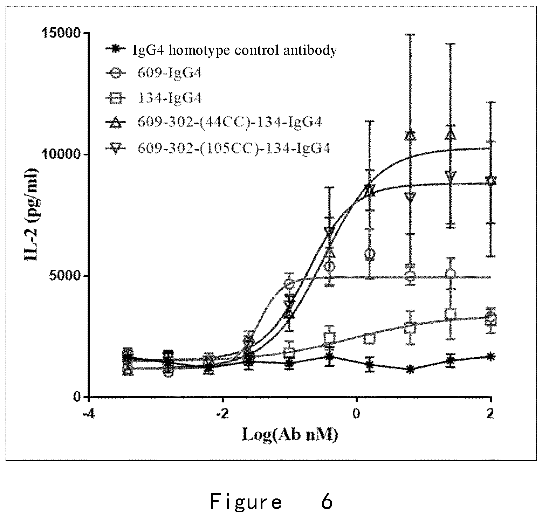

Figure 6 shows the ELISA results of the trispecific antibodies of the present invention.

### Detailed description

After extensive and in-depth research and extensive screening, the inventor successfully obtained for the first time a novel hexavalent trispecific antibody against PD-1, HER-2 and LAG-3. In the first aspect of the invention, a hexavalent trispecific antibody is provided. The hexavalent trispecific antibody is a dimer formed by two monomers, wherein each monomer comprises a heavy chain, a first light chain and a second light chain. Specifically, based on the structure of natural antibody, the structure of the first light chain and the heavy chain are designed and optimized, so that the trispecific antibody of the invention has similar or even better biological activity and physical and chemical properties than the monoclonal antibody while retaining the trispecificity of anti-PD-1, HER-2 and LAG-3. On this basis, the present invention has been completed.

### The terms

As used herein, the term "antibody(Ab)" or "immunoglobulin(IgG)" is a heterotetrameric glycoprotein with the same structural characteristics, which consists of two identical light chains (L) and two identical heavy chains (H). Each light chain is connected to the heavy chain through a covalent disulfide bond, and the numbers of disulfide bonds between heavy chains of different immunoglobulin isotypes are different. Each heavy and light chain also has regularly spaced intrachain disulfide bonds. Each heavy chain has a variable region (VH) at one end, followed by constant regions, which consists of three domains CH1, CH2, and CH3. Each light chain has a variable region (VL) at one end and a constant region at the other end; the constant region of the light chain is paired to the first constant region of the heavy chain, and the variable region of the light chain is paired to the variable region of the heavy chain. The constant regions are not directly involved in the binding of antibodies to antigens, but they exhibit different effector functions, such as participating in antibody-dependent cell-mediated cytotoxicity(ADCC). The heavy chain constant region includes IgG1, IgG2, IgG3, IgG4 subtypes; The light chain constant region includes κ (Kappa) or λ (Lambda). The heavy and light chains of the antibody are covalently linked together by disulfide bonds between the CH1 domain of the heavy chain and the CL domain of the light chain, and the two heavy chains of the antibody are covalently linked together by the polypeptide disulfide bonds formed between the hinge regions.

In the present invention, the term "trispecific antibody (or triantibody)" refers to an antibody molecule that can specifically bind three antigens (targets) or three epitopes simultaneously. Based on symmetry, trispecific antibodies can be divided into structurally symmetric and asymmetrical molecules. According to the number of binding sites, trispecific antibodies can be divided into trivalent, tetravalent, and multivalent molecules.

In the present invention, the term "monoclonal antibody (Mabs)" refers to an antibody obtained from a population that is essentially homogeneous, i.e. the population contains individual antibodies that are identical except for a few naturally occurring mutations that may be present. Monoclonal antibody targets a single antigen site with high specificity. Furthermore, unlike conventional polyclonal antibodies preparation, which are usually target antibodies against different determinants, each monoclonal antibodies targets a single determinant on an antigen. In addition to their specificity, the benefit of monoclonal antibodies is that they are synthesized by hybridoma culture and are not contaminated with other immunoglobulins. The modifier "monoclonal" indicates the properties of the antibody and is obtained from a homogeneous antibody population, which should not be interpreted as requiring any special method to produce the antibody.

"Humanized antibody" of the present invention refers to its CDR derived from non-human species (preferably mouse) antibody, the residual portion of the antibody molecule (including frame region and constant region) derived from human antibody. In addition, the framework residues can be altered to maintain binding affinity.

In the present invention, the terms "Fab" and "Fc" refer to papain may cleave the antibody into two identical Fab segments and one Fc segment. The Fab segment consists of VH and CH1 of the heavy chain of the antibody and VL and CL domains of the light chain. The Fab segment is fragment crystallizable (Fc), which consists of CH2 and CH3 domains of the antibody. The Fc segment has no antigen-binding activity and is the site of interaction between antibodies and effector molecules or cells.

In the present invention, the term "variable" means that certain parts of the variable region of an antibody differ in sequence, which forms the binding and specificity of various specific antibodies for their specific antigens. However, the variability is not evenly distributed throughout the variable region of the antibody. It is concentrated in three segments called complementary determining regions (CDRs) or hypervariable regions in the light chain and heavy chain variable regions. The more conserved part of the variable region is called the framework region (FR). The variable regions of the natural heavy and light chains each contain four FR regions, which are roughly in the β-folded configuration, connected by the three CDRs that form the connecting loop, and in some cases may form a partly β folded structure. The CDRs in each chain get close through the FR regions and together with the CDRs of the other chain form the antigen-binding site of the antibody (see Kabat et al., NIH Publ. No. 91-3242, Volume I, pages 647-669 (1991)).

As used herein, the term "framework region (FR)" refers to amino acid sequences inserted between CDRs, i.e., those portions of the relatively conserved light and heavy chain variable regions of immunoglobulins between different immunoglobulins in a single species. The light and heavy chains of immunoglobulins each have four FRs, which are called FR1-L, FR2-L, FR3-L, FR4-L and FR1-H, FR2-H, FR3-H, FR4-H. Accordingly, the light chain variable domain may thus be referred to as (FR1-L)-(CDR1-L)-(FR2-L)-(CDR2-L)-(FR3-L)-(CDR3-L)-(FR4-L) and the heavy chain variable domain may thus be represented as (FR1-H)-(CDR1-H)-(FR2-H)-(CDR2-H)-(FR3-H)-(CDR3-H)-(FR4-H). Preferably, the FR of the present invention is a human antibody FR or its derivatives, the derivative of the human antibody FR is substantially the same as the naturally occurring human antibody FR, i.e., sequence identity reaches 85%, 90%, 95%, 96%, 97%, 98% or 99%.

Knowing the amino acid sequence of the CDR, those skilled in the art can easily determine the frame region FR1-L, FR2-L, FR3-L, FR4-L and/or FR1-H, FR2-H, FR3-H, FR4-H.

As used herein, the term "human frame region" is substantially the same (about 85% or more, specifically 90%, 95%, 97%, 99% or 100%) framework region as the naturally occurring human antibody framework region.

As used herein, the term "linker" or "peptide linker" refers to one or more amino acid residues inserted into the immunoglobulin domain to provide sufficient mobility for the domain of light and heavy chains to fold into exchange for dual variable domain immunoglobulins. In the present invention, preferred linkers refer to linkers L1, L2, and L3, wherein L1 connects the heavy chain variable region of the first antibody to the heavy chain variable region of the second antibody, L2 connects the heavy chain variable region of the second antibody to the heavy chain variable region of the third antibody, and L3 connects the light chain variable region of the first antibody to the light chain variable region of the second antibody.

Examples of suitable linkers include monoglycine (Gly), or serine (Ser) residues, and the identification and sequence of amino acid residues in the linker may vary with the type of secondary structural element to be implemented in the linker.

### Trispecific antibody

The trispecific antibody of the present invention is a hexavalent trispecific antibody against PD-1, HER-2, and LAG-3, comprising an anti PD-1 antibody portion, an anti HER-2 antibody portion, and an anti LAG-3 antibody portion.

Preferably, the sequence of the anti PD-1 antibody of the present invention is as described in patent application WO 2018/137576 A1. Those skilled in the art can also modify or modify the anti PD-1 antibody of the present invention through well-known techniques in the art, such as adding, deleting, and/or replacing one or several amino acid residues, thereby further increasing the affinity or structural stability of the anti PD-1, and obtaining the modified or modified results through conventional measurement methods.

In the present invention, the trispecific antibody of the present invention also includes conservative variants thereof, which means that compared with the amino acid sequence of the antibody of the present invention, there are at most 10, preferably at most 8, more preferably at most 5, most preferably at most 3 amino acids replaced by amino acids with the same or similar properties to form a polypeptide. These conservatively variant polypeptides are preferably produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

In a preferred embodiment of the invention, the obtained antibody sequence information is shown in Table 1 below.

**Table 1 Sequence information Antibody of the present invention**

| SEQ ID NO: | Sequence name |
|---|---|
| 1 | 609-IgG4 heavy chain variable region amino acid sequence |
| 2 | 609-IgG4 light chain variable region amino acid sequence |
| 3 | 609-IgG4 heavy chain constant region amino acid sequence |
| 4 | 609-IgG4 light chain constant region amino acid sequence (i.e., human Kappa light chain constant region amino acid sequence) |
| 5 | 609-IgG4 heavy chain amino acid sequence |
| 6 | 609-IgG4 light chain amino acid sequence |
| 7 | 302-IgG1 heavy chain variable region amino acid sequence |
| 8 | 302-IgG1 light chain variable region amino acid sequence |
| 9 | Human IgG1 heavy chain constant region amino acid sequence |
| 10 | 302-IgG1 heavy chain amino acid sequence |
| 11 | 302-IgG1 light chain amino acid sequence |
| 12 | 134-IgG4 heavy chain amino acid sequence |
| 13 | 134-IgG4 light chain amino acid sequence |
| 14 | 609VH-302VH-134-IgG4 amino acid sequence |
| 15 | 609VL-302VL amino acid sequence |
| 16 | 609VH-302VH(44C)-134-IgG4 amino acid sequence |
| 17 | 609VL-302VL(100C) amino acid sequence |
| 18 | 609VH-302VH(105C)-134-IgG4 amino acid sequence |
| 19 | 609VL-302VL(43C) amino acid |

wherein, any of the above amino acid sequences also includes derivative sequences with PD-1, HER-2 and LAG-3 binding affinity that have been added, deleted, modified and/or replaced by at least one (e.g., 1-5, 1-3, preferably 1-2, preferably 1) amino acid.

In another preferred embodiment, the sequence with at least one amino acid added, deleted, modified and/or substituted in any of the above amino acid sequences is preferably an amino acid sequence having a homology of at least 80%, more preferably at least 85%, more preferably at least 90%, most preferably at least 95% to the above amino acid sequence.

In the present invention, the number of added, absent, modified, and/or substituted amino acids is usually 1, 2, 3, 4, or 5, preferably 1-3, more preferably 1-2, and most preferably 1.

In the present invention, preferred substitutions include, in the trispecific antibody 609VH-302VH-134-IgG4 of the present invention, the G at the 44th position of the variable region of the 302 heavy chain is replaced with C, and the Q at 100-position mutation of the variable region of the 302 light chain in the 609VL-302VL is mutated into C;

The Q mutation at position 105 of the variable region of the 302 heavy chain in 609VH-302VH-134-IgG4 is mutated to C, and the 43 position Q of the variable region of the light chain 302 in the 609VL-302VL was mutated to C.

In the present invention, the terms "anti", "binding and "specific binding" refers to a non-random binding reaction between two molecules, such as a reaction between an antibody and an antigen targeted thereto. Typically, antibody binds to this antigen with an equilibrium dissociation constant (KD) of less than about 10⁻⁷M, e.g. less than about 10⁻⁸M, 10⁻⁹M, 10⁻¹⁰M, 10⁻¹¹M, or less. In the present invention, the term "KD" refers to the equilibrium dissociation constant of a particular antibody-antigen interaction, which is used to describe the binding affinity between the antibody and the antigen. The smaller the equilibrium dissociation constant, the closer the antibody-antigen binding and the higher the affinity between the antibody and the antigen. For example, the binding affinity of an antibody to an antigen is determined using Surface Plasmon Resonance (SPR) in a BIACORE apparatus or the relative affinity of an antibody binding to an antigen is determined using an ELISA.

In the present invention, the term "valence" refers to the presence of a specified number of antigen binding sites in the antibody molecule. Preferably, the trispecific antibody of the present invention has six antigen-binding sites and is hexavalent. In the present invention, the antigen binding site comprises a heavy chain variable region (VH) and a light chain variable region (VL).

In the present invention, the term "epitope" refers to a peptide determinant specifically binding to an antibody. The epitope of the present invention is the region of the antigen bound by the antibody.

As used herein, the term "first light chain" refers to a light chain that contains a variable region of the light chain for antibodies against different targets. Specifically, the "first light chain" comprises a light chain variable region of an antibody targeting the first target and a light chain variable region of an antibody targeting the second target, which can be paired with a heavy chain variable region of an antibody targeting the first target and a heavy chain variable region of an antibody targeting the second target to form a first binding site that specifically binds the first target and a second binding site that specifically binds the second target.

Preferably, the "first light chain" in the present invention comprises two antibody light chain variable regions targeting different targets, either through a linker or directly connected. Furthermore, the "first light chain" in the present invention can be paired with the heavy chain at different positions through flexible linkers or bonds. Specifically, the "first light chain" in the present invention can pair with the heavy chain of a trispecific antibody through non covalent or covalent bonds. Preferably, the "first light chain" in the present invention pairs with the heavy chain through covalent bonds (such as disulfide bonds).

The trispecific antibodies of the present invention can be used alone or in combination with detectable markers (for diagnostic purposes), therapeutic agents, or any combination of these substances.

### Coding nucleic acid and expression vector

The present invention also provides the polynucleotide molecule encoding the antibody above mentioned or fragment thereof or fusion protein thereof. The polynucleotide of the present invention may be in the form of DNA or RNA. DNA forms include cDNA, genomic DNA, or synthetic DNA. DNA can be single stranded or double stranded. DNA may be a coding strand or a non-coding strand.

In the present invention, the term "expression vector" means a vector carrying an expression box for expressing a specific purpose protein or other substance, such as a plasmid, viral vector (e.g., adenovirus, retrovirus), phage, yeast plasmid, or other vector. Representative examples include but are not limited to: pTT5, pSECtag series, pCGS3 series, pcDNA series vectors, etc., and other vectors used for mammalian expression systems. The expression vector includes a fusion DNA sequence linked to a suitable transcriptional and translational regulatory sequence.

Once a relevant sequence is obtained, recombination methods can be used to obtain the relevant sequence in large quantities. This is usually carried out by cloning the sequence into a vector, transforming a cell with the vector, and then separating the relevant sequence from the proliferated host cell by conventional methods.

The present invention further relates to a vector comprising said suitable DNA sequence and a suitable promoter or a control sequence. These vectors can be used to transform suitable host cells to enable them to express protein.

In the present invention, the term "host cell" refers to cells suitable for expressing the above expression vector, which can be eukaryotic cells, such as mammalian or insect host cell culture systems can be used for the expression of fusion proteins of the present invention, CHO (Chinese hamster ovaries, Chinese Hamster Ovary), HEK293, COS, BHK and derived cells of the above cells can be applied to the present invention.

### Pharmaceutical Composition and Application

The present invention further provides a composition. Preferably, the composition is a pharmaceutical composition comprising the antibody above mentioned, or an active fragment thereof, or a fusion protein thereof, and a pharmaceutically acceptable carrier. Typically, these substances may be formulated in a non-toxic, inert and pharmaceutically acceptable aqueous carrier medium, wherein the pH is typically about 5-8 and preferably about 6-8, although the pH may vary depending on the nature of the substance being formulated and the condition to be treated. The formulated pharmaceutical composition may be administered by conventional routes, including (but not limited to): intravenous injection, intravenous drip, subcutaneous injection, local injection, intramuscular injection, intratumoral injection, intraperitoneal injection (e.g., intraperitoneal), intracranial injection, or intraluminal injection.

In the present invention, the term "pharmaceutical composition" refers to the hexavalent trispecific antibody of the present invention and a pharmaceutically acceptable carrier to form a pharmaceutical preparation composition so as to exert a more stable curative effect, and these preparations can ensure the conformational integrity of the amino acid core sequence of the antibody binding to human PD-1 or its antigen-binding fragment or hexavalent trispecific antibody disclosed in the present invention, and also protect the multifunctional groups of the protein from its degradation (including but not limited to coagulation, deamination or oxidation).

The pharmaceutical composition according to the present invention comprises a safe and effective amount (e.g. 0.001-99 wt %, preferably 0.01-90 wt %, preferably 0.1-80 wt %) of the hexavalent trispecific antibody above mentioned according to the present invention (or a conjugate thereof) and a pharmaceutically acceptable carrier or excipient. Such carriers include (but are not limited to): saline, buffers, glucose, water, glycerol, ethanol, and a combination thereof. Pharmaceutical preparations should correspond to the administration modes. The pharmaceutical composition according to the present invention can be prepared in the form of an injection, for example, by a conventional method using physiological saline or an aqueous solution containing glucose and other adjuvants. A pharmaceutical composition, for example, an injection and a solution, should be prepared under aseptic conditions. The dosage of the active ingredient is a therapeutically effective amount, for example, about 10 µg/kg body weight per day to about 50 mg/kg body weight. In addition, the hexavalent trispecific antibody of the present invention may also be used in combination with an additional therapeutic agent.

When a pharmaceutical composition is used, a safe and effective amount of an trispecific antibody or immunoconjugate thereof is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 10 mg per kilogram of body weight. Of course, a specific amount should also depend on the factors such as administration route and physical conditions of a patient, which fall into the skills of skilled physicians.

### Immunoconjugates

The present invention also provides an immunoconjugate based on the trispecific antibodies of the present invention.

Typically, the antibody-drug conjugate comprises the antibody and an effector molecule, wherein the antibody is conjugated to the effector molecule, and chemical conjugation is preferred. Preferably, the effector molecule is a therapeutically active drug. In addition, the effector molecule may be one or more of a toxic protein, a chemotherapeutic drug, a small-molecule drug or a radionuclide.

The trispecific antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker can be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is CH3COO-, Cl- or NO3-; and polymethylene dimethyl sulfide thiosulfonate. The linker may include, for example, a maleimide linked to an antibody via thiosuccimide.

A drug may be any cytotoxic, cytostatic or immunosuppressive drug. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA minor groove binding agents, DNA alkylating agents, and tubulin inhibitors; typical cytotoxic drugs include, for example, auristatins, camptothecins, docamycin/duocarmycins, etoposides, maytansines and maytansinoids (e.g. DM1 and DM4), taxanes, benzodiazepines or benzodiazepine containing drugs (e.g. pyrrolo[1,4]benzodiazepines (PBDs), indolinobenzodiazepines and oxazolidinobenzodiazepines), and vinca alkaloids.

In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in Bioconjugation Technology (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), binding an antibody to a drug-linker compound.

In certain embodiments, the method according to the present invention comprises: under conditions sufficient to form an antibody-linker conjugate, binding an antibody to a bifunctional linker compound. In these embodiments, the method according to the present invention further comprises: under conditions sufficient to covalently link the drug moiety to the antibody via a linker, binding the antibody-linker conjugate to the drug moiety.

In some embodiments, an antibody-drug conjugate (ADC) has a formula as follows: 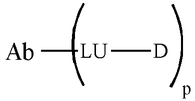 wherein,
Ab is an antibody,
LU is a linker;
D is a drug;
and the subscript p is a value selected from 1 to 8.

**The main advantages of the present invention include:**
(1) the present invention provides a hexavalent trispecific antibody with novel structure;
(2) The trispecific antibody of the present invention does not need to be modified by Fc, does not produce mismatch problem, and the preparation method is simple. (3) The trispecific antibody of the present invention retains the anti-PD-1, HER-2 and LAG-3 trispecifics, and has similar or even better biological activity and physicochemical properties than the monoclonal antibody.

The invention is further illustrated below in conjunction with specific embodiments. It should be understood that the examples are not intended to limit the scope of the invention. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

The protein expression and purification method used in the embodiment is described as follows: the target gene is constructed into the expression vector pcDNA3.4, and the constructed expression vector or a combination of expression vectors are transferred into FreeStyle^{™} 293-F Cells cells (hereinafter referred to as HEK293F, purchased from Thermo Fisher Scientific) by using PEI (Polyethylenimine) HEK293F cells were cultured in Free Style 293 Expression Medium (purchased from Thermo Fisher Scientific) for 5 days, cell supernatants were collected, and antibodies were purified by Protein A affinity chromatography.

The ELISA detection method used in the following embodiments is described as follows: the corresponding recombinant protein is coated on the microplate, and the microplate is closed with PBST containing 1% bovine serum albumin (PBST is a phosphate buffer containing 0.05% Tween-20). The antibody to be tested was sequentially diluted and then transferred to the above recombinant protein-coated microplates, incubated at room temperature for half an hour and then washed, appropriately diluted HRP (Horseradish Peroxidase)-labeled goat anti-human antibody (Fc specific, purchased from Sigma) was incubated at room temperature for half an hour and then washed, and 100 µl of TMB (3,3',5,5'-Tetramethylbenzidine) was added to each well. For the chromogenic solution of the substrate, the plate was incubated at room temperature for 1-5min, 50 µl of stop solution (2M H₂SO₄) was added to stop the reaction, the OD450 was read with a microplate reader (SpectraMax 190), GraphPad Prism7 was used for plotting and data analysis, and the EC50 was calculated.

The description of the physical and chemical property detection method used in the following embodiments is as follows:

### HPLC-SEC

Antibodies are high molecular weight proteins with highly complex secondary and tertiary structures. Due to post-translational modifications, aggregation, and degradation, antibodies exhibit heterogeneity in terms of biochemical and biophysical properties. When trispecific antibodies are analyzed by isolation techniques, variants, aggregates, and degraded fragments are often observed, and their presence may compromise safety and efficacy. During the production and storage of antibodies, aggregates, degraded fragments, and incomplete assembly of molecules are prone to occur. The present invention uses high-performance liquid chromatography size exclusion chromatography (HPLC-SEC) to detect the content of the above-mentioned impurities in the sample. The molecular weight of aggregates is greater than that of monomers, so the retention time of corresponding peaks is shorter; The molecular weight of degraded fragments or partially assembled molecules is smaller than that of monomers, therefore the retention time of corresponding peaks is longer. The chromatograph used for HPLC-SEC is Dionex Ultimate 3000; The preparation method for mobile phase is as follows: Take an appropriate amount of 20mM sodium dihydrogen phosphate mother liquor, and adjust the pH to 6.8 ± 0.1 with 20mM sodium dihydrogen phosphate; Injection volume: 20 µ g; The chromatographic column is TSK G3000SWXL, with a specification of 7.8 × 300mm 5 µ M; Flow rate of 0.5 ml/min, elution time of 30 minutes; Column temperature of 25 °C, sample room temperature of 10 °C; The detection wavelength is 214nm.

### HPLC-SEC

Many post-translational modifications, such as N-glycosylation, C-terminal lysine residue modification, N-terminal glutamine or glutamate cyclization, asparagine deamidation, aspartate isomerization, and amino acid residue oxidation, can directly or indirectly cause changes in the surface charge of antibodies, leading to charge heterogeneity. Charge variants can be separated and analyzed based on the charge charged, and the commonly used analytical methods are cation exchange chromatography (CEX) and anionexchange chromatography (AEX). When analyzed by a chromatography-based approach, acidic species and basic species are defined based on their retention time relative to the main peak. The acidic species is the variant that elutes earlier than the main peak of CEX or later than the main peak of AEX, while the alkaline species is the variant that elutes later than the main peak of CEX or earlier than the main peak of AEX. The peaks corresponding to the acidic and alkaline types are called acidic and alkaline peaks, respectively. Charge variants are easily generated during the production and storage of antibodies. The present invention uses high-performance liquid chromatography size exclusion chromatography (HPLC-SEC) to detect the content of the above-mentioned impurities in the sample. The chromatograph used for HPLC-IEC is Dionex Ultimate 3000; Mobile phase A: 20mM PB pH 6.3, mobile phase B: 20mM PB+200mM NaCl pH 6.3, the ratio of the two mobile phases mixed varies with time according to a pre-set program, with a flow rate of 1.0 ml/min; Chromatographic column: Thermo PropacTM WCX-10; Column temperature of 30 °C, sample room temperature of 10 °C; Injection volume: 20 µ g; Detection wavelength: 214nm.

### CE-SDS

CE-SDS(Capillary Electrophoresis-Sodium Dodecyl Sulfate) is used to analyze the content of degraded fragments or incompletely assembled molecules in a sample. CE is divided into non-reducing and reducing types, samples used for the former do not need to use reducing agent DTT to destroy the intramolecular disulfide bonds during denaturation, while samples used for the latter need to use reducing agent DTT to destroy the intramolecular disulfide bonds during denaturation. Non-reducing and reducing CE-SDS are denoted as NR-CE-SDS and R-CE-SDS, respectively. The Maurice CE-SDS analysis system used was purchased from ProteinSimple and equipped with a UV 214nm detector.

### Example 1 Anti-PD-L1/PD-L2 Trispecific Antibody

### Example 1.1 Sequence

mAb1-25-Hu (hereinafter referred to as 609-IgG4) is a humanized monoclonal antibody against PD-1, and its heavy and light chain amino acid sequences are derived from SEQ ID NO:8 AND SEQ ID NO:10 (i.e., SEQ ID NO: 5 and 6 in the present invention) in WO 2018/137576 A1. The amino acid sequences of the heavy chain variable region and light chain variable region of 609-IgG4 are shown in SEQ ID NO: 1 and 2. The constant region of the 609-IgG4 heavy chain is human IgG4 (SEQ ID NO: 3, the hinge region contains S228P mutation), and the constant region of the light chain is human Kappa (SEQ ID NO: 4). Here, the coding genes for the heavy and light chains of 609-IgG4 are named 609-IgG4 HC and 609-IgG4 LC, respectively. The 609-IgG4-HC and 609-IgG4-LC genes were constructed into pcDNA3.4 expression vectors, and the antibodies were expressed and purified after the combination of the two vectors, and the obtained antibodies were named 609-IgG4.
609-IgG4 heavy chain variable region amino acid sequence SEQ ID NO: 1: EVKLVESGGGLVQPGGSLRLSCAASGFAFSSYDMSWVRQAPGKRLEWVATISGGG 
RYTYYPDTVKGRFTISRDNAKNSHYLQMNSLRAEDTAVYFCASPYGGYFDVWGQ
GTLVTVSS
609-IgG4 light chain variable region amino acid sequence SEQ ID NO: 2: 
EIVLTQSPATLSLSPGERATLSCRASQSISNFLHWYQQKPGQAPRLLIKYASQSISGIP
ARFSGSGSGTDFTLTISSLEPEDFAVYFCQQSNSWPHTFGQGTKVEIK
609-IgG4 heavy chain constant region amino acid sequence (SEQ ID NO: 3): 
ASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKT
KPREEQFNSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTTPPVLDSD
GSFFLYSRLTVDKSRWQEGNVFSCSVMHEALHNHYTQKSLSLSLGK
609-IgG4 light chain variable region amino acid sequence (SEQ ID NO: 4):
609-IgG4 heavy chain amino acid sequence ( SEQ ID NO: 5): 
EVKLVESGGGLVQPGGSLRLSCAASGFAFSSYDMSWVRQAPGKRLEWVATISGGG
RYTYYPDTVKGRFTISRDNAKNSHYLQMNSLRAEDTAVYFCASPYGGYFDVWGQ
GTLVTVSSASTKGPSVFPLAPCSRSTSESTAALGCLVKDYFPEPVTVSWNSGALTSG
VHTFPAVLQSSGLYSLSSVVTVPSSSLGTKTYTCNVDHKPSNTKVDKRVESKYGPP
CPPCPAPEFLGGPSVFLFPPKPKDTLMISRTPEVTCVVVDVSQEDPEVQFNWYVDGV
EVHNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSLSLGK
609-IgG4 light chain amino acid sequence (SEQ ID NO: 6):

EIVLTQSPATLSLSPGERATLSCRASQSISNFLHWYQQKPGQAPRLLIKYASQSISGIP
ARFSGSGSGTDFTLTISSLEPEDFAVYFCQQSNSWPHTFGQGTKVEIKRTVAAPSVFI
FPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC

From published literature (Magdelaine-Beuzelin C, Kaas Q, Wehbi V, et al. Structure-function relationships of the variable domains of monoclonal antibodies approved for cancer treatment[J]. Critical reviews in oncology/hematology, 2007, 64(3):210-225.) The amino acid sequences of the heavy chain variable region and the light chain variable region of Trastuzumab (anti-HER-2 humanized monoclonal antibody) were obtained (SEQ ID NO: 7 and 8). The heavy chain variable region and light chain variable region are linked to the human IgG1 heavy chain constant region (SEQ ID NO: 9) and the human Kappa light chain constant region (SEQ ID NO: 4), respectively, to obtain full-length heavy and light chain amino acid sequences (SEQ ID NO: 10 and 11). Here, the genes encoding the full-length heavy and light chains were named 302-HC and 302-LC, respectively. The 302-IgG4-HC and 302-IgG4-LC genes were constructed into pcDNA3.4 expression vectors, and the antibodies were expressed and purified after the combination of the two vectors, and the obtained antibodies were named 609-IgG4.
302-IgG1 heavy chain variable region amino acid sequence ( SEQ ID NO: 7):
302-IgG1 light chain variable region amino acid sequence (SEQ ID NO: 8):
Human IgG1 heavy chain constant region amino acid sequence (SEQ ID NO: 9): 
ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPA
PELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLD
SDGSFFLYSKLTVDKSRWQQGNVFSCSVMHEALHNHYTQKSLSLSPGK
302-IgG1 heavy chain amino acid sequence (SEQ ID NO: 10): 
EVQLVESGGGLVQPGGSLRLSCAASGFNIKDTYIHWVRQAPGKGLEWVARIYPTNG
YTRYADSVKGRFTISADTSKNTAYLQMNSLRAEDTAVYYCSRWGGDGFYAMDYW
GQGTLVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSP
GK
302-IgG1 light chain amino acid sequence (SEQ ID NO: 11:

DIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIKRTVAAPS
VFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

134 Hu IgG4-C91S (hereinafter referred to as 134 IgG4) is a humanized monoclonal antibody against human LAG-3. Its heavy and light chain amino acid sequences are derived from SEQ ID NO: 32 and SEQ ID NO: 36 in WO 2020/173378 A1 (i.e. SEQ ID NO: 12 and 13 in the present invention). The heavy chain constant region of 134 IgG4 monoclonal antibody is human IgG4 (with S228P mutation in the hinge region), and the light chain constant region is human Kappa. Here, the coding genes for the heavy and light chains of 134-IgG4 are named 609-IgG4-HC and 609-IgG4-LC, respectively. The 134-IgG4-HC and 134-IgG4-LC genes were constructed into pcDNA3.4 expression vectors, and the antibodies were expressed and purified after the combination of the two vectors, and the obtained antibodies were named 134-IgG4.

Amino acid sequence of 134 humanized heavy chain 134-IgG4-HC (SEQ ID NO: 12):

QVQLVQSGAEVKKPGASVKVSCKASGYTLTAYYMNWVRQAPGQSLEWIGVINPY
NGDSSYNQKFKGRATLTVDKSTSTAYMELSSLRSEDTAVYYCARDDGYYRWYFD
VWGQGTLVTVSSASTKGPSVFPLAPCSRSTSESTAALGCLVKDYFPEPVTVSWNSG
ALTSGVHTFPAVLQSSGLYSLSSVVTVPSSSLGTKTYTCNVDHKPSNTKVDKRVES
KYGPPCPPCPAPEFLGGPSVFLFPPKPKDTLMISRTPEVTCVVVDVSQEDPEVQFNW
YVDGVEVHNAKTKPREEQFNSTYRVVSVLTVLHQDWLNGKEYKCKVSNKGLPSSI
EKTISKAKGQPREPQVYTLPPSQEEMTKNQVSLTCLVKGFYPSDIAVEWESNGQPE

NNYKTTPPVLDSDGSFFLYSRLTVDKSRWQEGNVFSCSVMHEALHNHYTQKSLSLS
LGK (The underlined part is the heavy chain complementarity decision area)

Amino acid sequence of 134 humanized light chain 134-IgG4-LC (SEQ ID NO: 13):

DIQMTQSPSSLSASVGDRVTITCRASQDIGSRLNWLQQKPGKSIKRLIYATSSLESGV
PSRFSGSRSGSDYTLTISSLQPEDFATYYCLQSGSSPPTFGQGTKVEIKRTVAAPSVFI
FPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC (The underlined part is
the light chain complementarity decision area).

### Example 1.2 Preparation of trispecific antibody

The heavy chain variable region of 609-IgG4 is connected to the heavy chain variable region of 302-IgG1 through artificial linkers (three tandem GGGGS), and then to the heavy chain of 134-IgG4 (the hinge region contains S228P mutation) through artificial linkers (three tandem GGGGS). The long heavy chain gene containing three heavy chain variable regions is named as 609VH-302VH-134-IgG4 (SEQ ID NO: 14). The light chain variable region of 609 is connected to the light chain variable region of 302 IgG1 through artificial linkers (three tandem GGGGS). The long light chain gene containing two light chain variable regions constructed through this program is named as 609VL-302VL (SEQ ID NO: 15).

The encoding genes of the above sequences were constructed into pcDNA3.4 expression vectors, and the expression vectors of 609VH-302VH-134-IgG4, 609VL-302VL and 134-IgG4-LC were combined to express and purify the antibodies, and the obtained antibodies were named as 609-302-134-IgG4.
609VH-302VH-134-IgG4 amino acid sequence 
EVKLVESGGGLVQPGGSLRLSCAASGFAFSSYDMSWVRQAPGKRLEWVATISGGG
RYTYYPDTVKGRFTISRDNAKNSHYLQMNSLRAEDTAVYFCASPYGGYFDVWGQ
GTLVTVSSGGGGSGGGGSGGGGSEVQLVESGGGLVQPGGSLRLSCAASGFNIKDTY
IHWVRQAPGKGLEWVARIYPTNGYTRYADSVKGRFTISADTSKNTAYLQMNSLRA
EDTAVYYCSRWGGDGFYAMDYWGQGTLVTVSSGGGGSGGGGSGGGGSQVQLVQ
SGAEVKKPGASVKVSCKASGYTLTAYYMNWVRQAPGQSLEWIGVINPYNGDSSYN
QKFKGRATLTVDKSTSTAYMELSSLRSEDTAVYYCARDDGYYRWYFDVWGQGTL
VTVSSASTKGPSVFPLAPCSRSTSESTAALGCLVKDYFPEPVTVSWNSGALTSGVHT
FPAVLQSSGLYSLSSVVTVPSSSLGTKTYTCNVDHKPSNTKVDKRVESKYGPPCPPC
PAPEFLGGPSVFLFPPKPKDTLMISRTPEVTCVVVDVSQEDPEVQFNWYVDGVEVH
NAKTKPREEQFNSTYRVVSVLTVLHQDWLNGKEYKCKVSNKGLPSSIEKTISKAKG
QPREPQVYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLGK (SEQ ID
NO: 14)
609VL-302VL amino acid sequence:

EIVLTQSPATLSLSPGERATLSCRASQSISNFLHWYQQKPGQAPRLLIKYASQSISGIP
ARFSGSGSGTDFTLTISSLEPEDFAVYFCQQSNSWPHTFGQGTKVEIKGGGGSGGGG
SGGGGSDIQMTQSPSSLSASVGDRVTITCRASQDVNTAVAWYQQKPGKAPKLLIYS

ASFLYSGVPSRFSGSRSGTDFTLTISSLQPEDFATYYCQQHYTTPPTFGQGTKVEIK
(SEQ ID NO: 15)

In order to enhance the stability of the trispecific antibody, an attempt was made to introduce a pair of disulfide bonds between 609VH-302VH-134-IgG4 and 609VL-302VL. According to the reference (Cho, Hyun-so & Mason, Karen & Ramyar, Kasra & Stanley, Ann & Gabelli, Sandra & Denney, Dan & Leahy, Daniel. (2003). Structure of the extracellular region of HER2 alone and in complex with the Herceptin Fab. Nature. 421. 756-60. 10.1038/nature01392.) The crystal structure of Trastuzumab (see https://www.rcsb.org/structure/1N8Z for related information) is reported here, in which pairs of spatially similar amino acid residues at the interface between VH and VL are selected and mutated into cysteine: amino acid at position 44 on VH and amino acid at position 100 on VL, amino acid at position 105 on VH and amino acid at position 43 on VL (The amino acid residues described above are encoded according to Kabat's rules).

Here, the G at the position 44 of the variable region of the 302 heavy chain in 609VH-302VH-134-IgG4 was mutated into C, and the obtained sequence was named 609VH-302VH(44C)-134-IgG4, and Q at the position 100 of the variable region of the 302 light chain in 609VL-302VL was mutated to C, and the obtained sequence was named 609VL-302VL(100C);
609VH-302VH(44C)-134-IgG4 amino acid sequence 
EVKLVESGGLVQPGGSLRLSCAASGFAFSSYDMSWVRQAPGKRLEWVATISGGGR
YTYPDTVKGRFTISRDNAKNSHYLQMNSLRAEDTAVYFCASPYGGYFDVWGQGTL
VTVSGGGGGGGGSEVQLVESGGLVQPGGLRLSCAASGFUNIKDTYIHWVRQAPGL
EWVARIYPTNGYTRYADSVKGRFTISADDTSKNTAYLQMNSLRAEDTAVYYCSCR
GGDGFYAMDY WGQGTLVTVSGGG
GSGGGGGGGSQVVQLVQSGAEVKKGASVKVSKASKYTLTAYYMNWVRQAPGQ
SLEWIGVINPYNGDSSYNQKFGRATTVDKSTAYMELSSLRSEDTAVYYCARDDDG
YYRWYFDVWGQGTLVTVSSASTKGPSVFPLA
PCSRSTSETAALGCLVKDYFPEPVTVSWNSGALTSVHTFPAVLQSSGLYLSSVVTVP
SSSLGTKTYTCNVDHKPSNTKVDKRVESKYGPCPPAPEFLGGPSVFPPKDTLMISRT
PEVTCVVDVSQEDPEVQFNWYVDGVEVHNAKTKPREEQFNSTYRVVSVLTVLTHQ
DNGKEYKCKVSNKGLPSSIEKTISKAKQPREPQVYTLPSQEEMTKNQVSLTCLVKG
FYPSDIAVEWESNGG GQP
ENNYKTTPVLDSDGSFLYSRLTVDKSRWQEGNVFSCSVMHEALHNHYTQKSLSLS
LGK (SEQ ID NO: 16, where the underline represents the mutation site)
609VL_302VL(100C) amino acid sequence 
EIVLTQSPATLSLSPGERATLSCRASQSISNFLHWYQQKPGQAPRLLIKYASQSISGIP
ARFSGSGSGTDFTLTISSLEPEDFAVYFCQQSNSWPHTFGQGTKVEIKGGGGSGGGG
SGGGGSDIQMTQSPSSLSASVGDRVTITCRASQDVNTAVAWYQQKPGKAPKLLIYS
ASFLYSGVPSRFSGSRSGTDFTLTISSLQPEDFATYYCQQHYTTPPTFGCGTKVEIK
(SEQ ID NO: 17, where the underlined is the mutation site)
 (SEQ ID NO: 17, where the underlined is the mutation site)

Here, the Q at the position 105 of the variable region of the 302 heavy chain in 609VH-302VH-134-IgG4 was mutated into C, and the obtained sequence was named 609VH-302VH(105C)-134-IgG4, and A at the position 43 of the variable region of the 302 light chain in 609VL-302VL was mutated to C, and the obtained sequence was named 609 VL-3 02 VL(43 C).
609VH-302VH(105C)-134-IgG4 amino acid sequence: 
EVKLVESGGLVQPGGSLRLSCAASGFAFSSYDMSWVRQAPGKRLEWVATISGG
GRYTYPDTVKGRFTISRDNAKNSHYLQMNSLRAEDTAVYFCASPYGYFDVWGQGT
LVTVSGGGGGGGGSEVQLVESGGLVQPGGLRLSCAASGFUNIKDTYIHWVRQAPG
KGLEWVARIYPTNGYTRYADSVKGRFTISADDTSKNTAYLQMNSLRAEDTAVYYC
SCRGGDGFYAMDY WGCGTLVTVSSGGG
GSGGGGGGGGGSQVVQLVQSGAEVKKPGKVSKASKYTLTAYYMNWVRQAPGQ
SLEWIGWINPYNGDSSYNQFKFGRATTVDKSTATTAYMELSSLRSEDTAVYYCARD
DDGYYRWYFDVWGQGTLVTVSSASTKGPSVFPLA
PCSRSTSETAALGCLVKDYFPEPVTVSWNSGALTSVHTFPAVLQSSGLYLSSVVTVP
SSSLGTKTYTCNVDHKPSNTKVDKRVESKYGPCPPAPEFLGGPSVFPPKDTLMISRT
PEVTCVVDVSQEDPEVQFNWYVDGVEVHNAKTKPREEQFNSTYRVVSVLTVLTHQ
DNGKEYKCKVSNKGLPSSIEKTISKAKQPREPQVYTLPSQEEMTKNQVSLTCLVKG
FYPSDIAVEWESNGG GQP
ENNYKTTPVLDSDGSFLYSRLTVDKSRWQEGNVFSCSVMHEALHNHYTQKSLSLS
LGK (SEQ ID NO: 18, where the underline represents the mutation site)
609VL-302VL(43C) amino acid sequence 
EIVLTQSPATLSLSPGERATLSCRASQSISNFLHWYQQKPGQAPRLLIKYASQSISGIP
ARFSGSGSGTDFTLTISSLEPEDFAVYFCQQSNSWPHTFGQGTKVEIKGGGGSGGGG
SGGGGSDIQMTQSPSSLSASVGDRVTITCRASQDVNTAVAWYQQKPGKCPKLLIYS
ASFLYSGVPSRFSGSRSGTDFTLTISSLQPEDFATYYCQQHYTTPPTFGQGTKVEIK
 (SEQ ID NO: 19, where the mutation site is underlined)

The coding genes of the above sequences were constructed into pcDNA3.4 expression vectors, and the corresponding vectors were combined (609VH-302VH-134-IgG4, 609VL-302VL and 134-IgG4-LC combinations). 609VH-302VH(44C)-134-IgG4, 609VL-302VL(100C) and 134-IgG4-LC combination, 609VH-302VH(105C)-134-IgG4, 609VL-302VL(43C) and 134-IgG4-LC combination) expressed and purified antibodies. The resulting antibodies were designated as 609-302-134-IGG4, 609-302-(44CC) -134-IGG4, and 609-302-(105CC) -134-IGG4, respectively.

The name of the antibody in the invention is shown in Table 2:

**Table 2 The antibody name of the invention**

| Antibody | name | Heavy chain | Light chain |
|---|---|---|---|
| Anti-PD-1 antibody | 609-IgG4 | 609-IgG4-HC | 609-IgG4-LC |
| Anti-HER-2 antibody | 302-IgG1 | 302-HC | 302-LC |
| Anti-LAG-3 antibody | 134-IgG4 | 134-IgG4-HC | 134-IgG4-LC |
| Trispecific antibodies against PD-1, HER-2 and LAG-3 | 609-302-134-Ig G4 | 609VH-302VH-134-Ig G4 | 609VL-302VL |
| | 609-302-(44CC) -134-IgG4 | 609VH-302VH(44C)-1 34-IgG4 | 609VL-302VL(100C) |
| | 609-302-(105C C)-134-IgG4 | 609VH-302VH(105C)-134-IgG4 | 609VL-302VL(43C) |

### Example 1.3 ELISA determination of relative affinity

Hereby, HER2 extracellular segment recombinant protein (referred to as HER2-His, purchased from Sino Biological Inc.), PD-1 extracellular segment recombinant protein (referred to as PD1-His, purchased from Sino Biological Inc.), and LAG-3 extracellular segment recombinant protein (referred to as LAG3-His, purchased from ACROBiosystems) with polyhistidine tags were coated on microplates at concentrations of 20ng/well, 20ng/well, and 10ng/well, respectively.

**Table 3. EC50 results for ELISA determination of relative affinity**

| **Antibody** | **EC₅₀ (nM)** | | |
|---|---|---|---|
| | **PD-1** | **HER-2** | **LAG-3** |
| 609-IgG4 | 0.1085 | NA | NA |
| 302-IgG1 | NA | 0.08564 | NA |
| 134-IgG4 | NA | NA | 0.122 |
| 609-302-134-IgG4 | 0.1251 | 0.4918 | 0.2598 |
| 609-302-(44CC)-134-IgG4 | 0.09111 | 0.3549 | 0.226 |
| 609-302-(105CC)-134-IgG4 | 0.104 | 0.4196 | 0.2865 |

As shown in Figure 2A-C, 609-302-134-IGG4, 609-302-(44CC) -134-IGG4 and 609-302-(105CC) -134-IGG4 can effectively bind PD-1, HER-2 and LAG-3, indicating that they are trispecific antibodies. EC50 is summarized in Table 3.

### Example 1.4 Characterization of physical and chemical properties

### 1.4.1 HPLC-SEC

Figure 3A shows the HPLC-SEC spectrum of monoclonal antibody 609-IgG4, with the main peak accounting for 99.87%.

Figure 3B shows the HPLC-SEC spectrum of 609-302-134 IgG4, with the main peak accounting for 98.24%.

Figure 3C shows the HPLC-SEC spectrum of 609-302- (44CC) -134-IgG4, with the main peak accounting for 98.4%.

Figure 3D shows the HPLC-SEC spectrum of monoclonal antibody -134-IgG4, with the main peak accounting for 98.06%.

The above results showed that the SEC purity of the trispecific antibody could reach more than 98% after purification by one-step Protein A affinity chromatography, and the size heterogeneity was close to that of the monoclonal antibody.

### 1.4.2 HPLC-IEC

Figure 4A shows the HPLC-SEC spectrum of monoclonal antibody 609-IgG4, with the main peak accounting for 71.45%.

Figure 4B shows the HPLC-IEC spectrum of 609-302-(44CC) -134-IGG4, with the main peak accounting for 71.62%.

Figure 4C shows the HPLC-IEC spectrum of 609-302-(105CC) -134-IGG4, with the main peak accounting for 66.12%.

The above results showed that the IEC main peak of the trispecific antibody could reach more than 66% after purification by one-step Protein A affinity chromatography, and the charge heterogeneity was comparable to that of monoclonal antibody.

### 1.4.3 CE-SDS

Figures 5A and 5B represent the NR-CE-SDS and R-CE-SDS spectra of monoclonal antibody 609-IgG4, respectively. In the NR-CE-SDS spectrum, the main peak Peak2.672 accounts for 98.9%; In the R-CE-SDS spectrum, the two main peaks Peak1.499 (corresponding to the light chain) and Peak1.888 (corresponding to the heavy chain) account for 31.3% and 67.7%, respectively. The sum of the two main peaks accounts for 99.0%.

Figures 5C and 5D represent the NR-CE-SDS and R-CE-SDS spectrum of 609-302-134 IgG4, respectively. The two main peaks Peak1.525 and Peak2.893 in the NR-CE-SDS spectrum account for 18.6% and 80.8%, respectively. In the 609-302-134 IgG4 molecule, 609VL-302VL does not form a covalent disulfide bond with 609VH-302VH-134-IgG4. Therefore, it is believed that the peptide chain corresponding to Peak1.525 is 609VL-302VL; In the R-CE-SDS spectrum, the two main peaks Peak1.518 (corresponding to the light chain) and Peak2.158 (corresponding to the heavy chain) account for 34.8% and 62.5%, respectively. The sum of the two main peaks accounts for 97.3%.

Figures 5E and 5F represent the NR-CE-SDS and R-CE-SDS spectrum of 609-302-(44CC) -134-IgG4, respectively, with the main peak Peak3.018 accounting for 96.4% in the NR-CE-SDS spectrum; In the R-CE-SDS spectrum, the two main peaks Peak1.515 (corresponding to the light chain) and Peak2.159 (corresponding to the heavy chain) account for 34.9% and 62.0%, respectively. The sum of the two main peaks accounts for 96.9%. Figures 5G and 5H represent the NR-CE-SDS and R-CE-SDS spectrum of 609-302-(105CC) -134-IGG4, respectively. In the NR-CE-SDS spectrum, the main peak Peak3.020 accounted for 91.8%. The two main peaks Peak1.508(corresponding to light chain) and Peak2.147(corresponding to heavy chain) accounted for 36.6% and 56.9% respectively in the R-CE-SDS spectrum, and the sum of the two main peaks accounted for 93.5%.

The above results show that the purity of NR-CE-SDS and R-CE-SDS of trispecific antibody 609-302-(44CC) -134-IGG4 could reach more than 96% after purification by one-step Protein A affinity chromatography, which was most similar to monoclonal antibody 609-IgG4.

### Example 2 The determination of ELISA results of the trispecific antibodies of the present invention.

RPMI 1640 was supplemented with the following additives: 10% fetal bovine serum, 1% MEM Non-Essential Amino Acids Solution, 1% Sodium Pyruvate, 1% HEPES, 1‰ 2-Mercaptoethanol, 1% Penicillin-Streptomycin, 1% GlutaMAX (media and supplements were purchased from Thermo Fisher Scientific). Freshly isolated human peripheral blood mononuclear cells (PBMCs) were cultured using the RPMI 1640 complete medium mentioned above. PBMC purchased from Allcells, item number: PB005-C) was washed and resuspended, and a certain amount of superantigen Staphylococcal Enterotoxin B (SEB) was added. SEB was prepared internally in the laboratory, and the sequence was obtained from Uniprot (Entry: P01552), expressed using E. coli, purified by Ni-NTA affinity chromatography. PBMC cell suspension was inoculated into 96-well cell culture plates with 150µl suspension and 200,000 cells per well. A 50µl gradient diluted related antibody was added to the 96-well plate. The 96-well plates were incubated in a cell incubator at 37°C for 4 days. An appropriate amount of cell culture supernatant from a 96 well plate was taken. IL-2 in the supernatant was detected by double-antibody sandwich ELISA (the paired antibody for the related assay was purchased from BD Biosciences). OD450 was read by SpectraMax 190, and EC50 was calculated by GraphPad Prism7.

**Table 4. Functional activity parameters of the trispecific antibody of the invention**

| **Antibody** | **EC₅₀ (nM)** | **Top** |
|---|---|---|
| 609-IgG4 | 0.04091 | 5363 |
| 134-IgG4 | 0.8398 | 3369 |
| 609-302-(44CC)-134-IgG4 | 0.3217 | 10281 |
| 609-302-(105CC)-134-IgG4 | 0.1834 | 8795 |

As shown in Figure 6 and Table 4, 609-IgG4 exhibits a smaller EC50 and a higher Top than 134-IgG4, indicating that 609-IgG4 is more functionally active than 134-IgG4. 609-302- (44CC) -134-IgG4 and 609-302- (105CC) -134-IgG4 exhibit similar EC50 and Top, indicating that the functional activity of these two trispecific antibodies is equivalent. Compared with 609-IgG4, 609-302- (44CC) -134-IgG4 and 609-302- (105CC) -134-IgG4 showed a higher Top, and their ability to stimulate PBMC to secrete IL-2 was significantly stronger than that of monoclonal antibodies 609-IgG4 and 134 IgG4 when the antibody concentration was greater than 1nM (i.e. log value greater than 0).

All publications mentioned herein are incorporated by reference as if each individual document was cited as a reference in the present application. It should be understood that, after reading the above teachings of the present invention, those skilled in the art can make various modifications and changes. These equivalent forms are also within the scope defined by the claims appended hereto.

## Claims

1. A hexavalent trispecific antibody, wherein the hexavalent trispecific antibody is a dimer formed by two monomers, and each monomer comprises a heavy chain, a first light chain and a second light chain;
the heavy chain comprises an antibody heavy chain variable region element Z1 for a first target, an antibody heavy chain variable region element Z2 for a second target, an antibody heavy chain variable region element Z3 for a third target, and a antibody heavy chain constant region Z4 in tandem from the N end to the C end;
the first light chain and the second light chain respectively cooperate with the heavy chain, so that the hexavalent trispecific antibody specifically binds to the first target, second target, and third target.

2. The hexavalent trispecific antibody of claim 1, wherein the antibody comprises a heavy chain, a first light chain, and a second light chain; and each heavy chain has the structure of formula I:
Z1-Z2-Z3-Z4 (I)
Z1 is a heavy chain variable region element for the first target;
Z2 is a heavy chain variable region element for the second target;
Z3 is a heavy chain variable region element for the third target;
Z4 is the constant heavy chain region CH1, CH2 and CH3;
"-" is independently a bond or a linker;
wherein the first light chain and the second light chain cooperate with the heavy chain respectively, so that the hexavalent trispecific antibody specifically binds to the first target, the second target and the third target, and the first light chain has the structure of the following formula II:
Z5-Z6 (II);
wherein Z5 is a light chain variable region element for the first target; Z6 is a light chain variable region element for the second target;
the second light chain has a structure shown in formula III:
Z7-Z8 (III);
wherein Z7 is a light chain variable region element for the third target, and Z8 is a light chain constant region.

3. The hexavalent trispecific antibody of claim 1 or 2, wherein the first target, the second target, and the third targe is PD-1, HER-2 and LAG-3.

4. The hexavalent trispecific antibody of any one of claims 1-3, wherein the linker is a peptide linker of 1-35 amino acids in length, and preferably a peptide linker of 6-30 amino acids in length.

5. The hexavalent trispecific antibody of any one of claims 1-4, wherein the hexavalent trispecific antibody comprises two monomers, each containing a heavy chain, a first light chain, and a second light chain, and each monomer has the structure of formula IV: 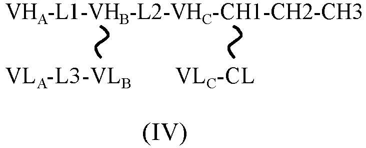 wherein,
VH_{A}-L1-VH_{B}-L2-VH_{C}-CH1-CH2-CH3 is the heavy chain;
VL_{A}-L3-VL_{B} is the first light chain;
VLc-CL is the second light chain;
VH_{A} is the heavy chain variable region of an anti-PD-1 antibody;
VH_{B} is the heavy chain variable region of an anti-HER-2 antibody;
VHc is the heavy chain variable region of an anti-LAG-3 antibody;
CH1, CH2 and CH3 are the constant heavy chain regions CH1, CH2 and CH3, respectively;
VL_{A} is the light chain variable region of an anti-PD-1 antibody;
VL_{B} is the light chain variable region of an anti-HER-2 antibody;
VLc is the light chain variable region of an anti-LAG-3 antibody;
CL is the light chain constant region of the antibody;
L1, L2, and L3 are independently linkers;
each "-" is independently a bond;
"~" represents a disulfide bond or covalent bond;
wherein the hexavalent trispecific antibody binds simultaneously to PD-1, HER-2 and LAG-3.

6. The hexavalent trispecific antibody of any one of claims 1-5, wherein the linker is a flexible peptide linker, and the flexible peptide linker comprises 6-30 amino acids, preferably 10-25 amino acids.

7. The hexavalent trispecific antibody of any one of claims 1-6, wherein the hexavalent trispecific antibody comprises two heavy chains, two first light chains and two second light chains,
the amino acid sequences of the heavy chain, the first light chain and the second light chain in the hexavalent trispecific antibody are shown as SEQ ID No: 14, 15 and 13, respectively; or
the amino acid sequences of the heavy chain, the first light chain and the second light chain in the hexavalent trispecific antibody are shown as SEQ ID No: 16, 17 and 13, respectively; or
the amino acid sequences of the heavy chain, the first light chain and the second light chain in the hexavalent trispecific antibody are shown as SEQ ID No: 18, 19 and 13, respectively.

8. An isolated nucleic acid molecule, wherein the nucleic acid molecule encodes a hexavalent trispecific antibody of any one of claims 1-7.

9. An express vector comprising the nucleic acid molecule of claim 8.

10. A host cell comprising the expression vector of claim 9.

11. A method for preparing the antibody of any one of claims 1-7, which comprises following steps:
(a) culturing the host cell of claim 10 to express the hexavalent trispecific antibody;
(b) separating and purifying the hexavalent trispecific antibody described in (a).

12. A pharmaceutical composition comprising the hexavalent trispecific antibody according to any one of claims 1-7 and a pharmaceutically acceptable carrier.

13. Use of the hexavalent trispecific antibody of any one of claims 1-7 or pharmaceutical composition of claim 12 in the preparation of drugs for the treatment of a cancer.

14. The use of claim 13, wherein the cancer is selected from the group consisting of:
melanoma, renal cancer, prostate cancer, pancreatic cancer, breast cancer, colon cancer, lung cancer, esophageal cancer, head and neck squamous cell cancer, liver cancer, ovarian cancer, cervical cancer, thyroid cancer, glioblastoma, glioma and other vegetative malignant diseases.

15. An immunoconjugate, wherein the immunoconjugate comprises:
(a) the hexavalent trispecific antibody of any one of claims 1-7; and
(b) a coupling moiety selected from the group consisting of a detectable label, drug, toxin, cytokine, radionuclide, or enzyme.
